Europäisches Patentamt

European Patent Office

Office européen des brevets

(19)

(11) Veröffentlichungsnummer: **0 212 144 B1**

# EUROPÄISCHE PATENTSCHRIFT

(45) Veröffentlichungstag der Patentschrift: **21.08.91**

(51) Int. Cl.⁵: **A61K 6/10**

(21) Anmeldenummer: **86108700.5**

(22) Anmeldetag: **26.06.86**

(54) **Dental Silikon-Abformmasse.**

(30) Priorität: **13.07.85 DE 3525054**

(43) Veröffentlichungstag der Anmeldung:
**04.03.87 Patentblatt 87/10**

(45) Bekanntmachung des Hinweises auf die
Patenterteilung:
**21.08.91 Patentblatt 91/34**

(84) Benannte Vertragsstaaten:
**AT BE CH DE FR GB IT LI LU NL SE**

(56) Entgegenhaltungen:
**EP-A- 0 016 988**
**DE-A- 1 802 374**
**FR-A- 1 261 155**
**FR-A- 2 076 293**

(73) Patentinhaber: **Blendax GmbH**
**Rheinallee 88**
**W-6500 Mainz 1(DE)**

(72) Erfinder: **Kuhlmann, Werner, Dr.**
**Vogelsbergerstrasse 11**
**W-6500 Mainz-Hechtsheim(DE)**
Erfinder: **Ott, Heidrun geb. Betzold**
**Am Weinberg 22**
**W-6200 Wiesbaden-Auringen(DE)**

EP 0 212 144 B1

**Beschreibung**

Die vorliegende Erfindung betrifft eine dentale Silikon-Abformmasse auf Basis von additions- oder kondensationsvernetzenden Silikonen mit verbesserten Gebrauchseigenschaften.

Dentale Abformmassen auf Basis additions- oder kondensationsvernetzender Silikone liegen in der Regel in Form von Pasten vor und enthalten, neben den beim Zusammenbringen mit dem Co-System polymerisierenden oder kondensierenden Organosilikonen in der Regel anorganische Füllstoffe sowie ggf. weitere Zusätze wie Thixotropiermittel. Das Co-System enthält Katalysatoren und Vernetzer, die beim Zusammenbringen mit der Basiszusammensetzung eine Aushärtung derselben bewirken.

Um die optimalen Abformeigenschaften aufweisen zu können, ist es erforderlich, daß beide Komponenten exakt entsprechend den Herstellvorschriften gemischt werden.

Um die genaue Dosierung zu erleichtern und sicherzustellen, geschieht dies in der zahnärztlichen Praxis üblicherweise auf einem Mischblock, auf dem die einzuhaltenden Stranglängen gekennzeichnet sind. Dabei tritt in der Regel die Schwierigkeit auf, daß die Einhaltung dieser Dosierungsvorschriften deshalb schwierig ist, weil die Abformmassen aufgrund ihrer Viskosität entweder verlaufen oder, bei einer hochviskosen Einstellung, die Fließfähigkeit der Masse so beeinträchtigt ist, daß die Handhabung erheblich erschwert wird.

Es bestand daher die Aufgabe, eine dentale Abformmasse auf Basis eines Organosilikons zu entwikkeln, die ein optimales, die genaue Dosierung nicht behinderndes Viskositätsverhalten aufweist.

Die erfindungsgemäße Lösung dieser Aufgabe besteht darin, einer solchen dentalen Abformmasse ein Aluminiummono-, -di- oder -trisalz einer Fettsäure mit 12 bis 18 C-Atomen zuzusetzen, vorzugsweise in einer Menge von 0,1 bis 10 Gew.-%, bezogen auf die Gesamtzusammensetzung, insbesondere 0,5 bis 5 Gew.-% der Abformmasse.

Als bevorzugtes Aluminiumsalz findet dabei Aluminiummono-, -di- oder tristearat Verwendung, wobei jedoch auch Gemische aller drei Salze eingesetzt werden können.

Ebenfalls geeignet sind die entsprechenden Aluminiumsalze der Laurin-,Myristin- oder Palmitinsäure; es können jedoch auch entsprechende Aluminiumsalze von Mischfettsäuren Verwendung finden.

Durch den Zusatz dieser Aluminiumsalze, insbesondere derjenigen der Stearinsäure, erhält die Abformmasse eine ausgeprägte Strukturviskosität, die ein exaktes Dosieren auf dem Mischblock ohne Verlaufen erlaubt, andererseits aber beim Anmischen mit der Härterkomponente durch die dabei angewandte Scherkraft so dünnflüssig wird, daß feinzeichnende Abformungen möglich sind.

Ein weiterer Vorteil der erfindungsgemäßen dentalen Abformmassen gegenüber den aus dem Stand der Technik bekannten thixotropen Systemen liegt darin, daß sie nach vorangegangener starker Scherbeanspruchung, wie sie beispielsweise im Dosierzylinder einer Abfüllmaschine auftritt, zum Wiederaufbau der Viskosität fähig sind.

Die Anwendung der erfindungsgemäß zusammengesetzten dentalen Abformmassen hat sich insbesondere bei der Herstellung von Korrekturabdrücken als vorteilhaft erwiesen.

Die in dentalen Abdruckmassen zum Einsatz gelangenden additions- oder kondensationsvernetzenden Organosilikone sind bekannt.

Es handelt sich bei der erstgenannten Gruppe vorwiegend um Vinylgruppen enthaltende Polydimethylsiloxane mit Hydrogenpolydimethylsiloxanen.

Hierbei ist zur Aushartung die Anwesenheit eines Katalysators erforderlich, wobei insbesondere platinhaltige Katalysatoren, Rhodiumverbindungen oder Metallcarbonyle wie Kobalt- und Mangancarbonyl verwendet werden können.

Bevorzugt sind platinhaltige Katalysatoren.

Kondensationsvernetzende Organosilikone sind Hydroxylgruppen enthaltende Polydiorganosiloxane. Diese können mit Alkyl-, Cycloalkyl- und Arylgruppen substituiert sein. Da diese Stoffe dem Fachmann geläufig sind (vgl. beispielsweise EP-A 16 988), erübrigt sich hier eine detaillierte Aufzählung.

Die aus kondensationsvernetzenden Organosilikonen bestehende, einen anorganischen Füllstoff enthaltende Phase wird vor Gebrauch durch Reaktion mit einer Katalysator-Vernetzermischung ausgehärtet.

Auch diese Vernetzer sind wohlbekannt, in der Regel handelt es sich dabei um O-Kieselsäure- und Polykieselsäureester; es können auch Silanvernetzungsmittel zugesetzt werden.

Da die Vernetzungsreaktion stets in Gegenwart eines Katalysators stattfindet, muß ein solcher der den Vernetzer enthaltenden Phase zugesetzt werden.

Geeignete Katalysatoren sind Metallsalze gegebenenfalls alkylsubstituierter Carbonsäuren, insbesondere Dibutylzinndilaurat, Dibutylzinndiacetat, Dioctylzinndi(2-ethylhexylhexanoat), oder auch Tetrabutyltitanat, Bleilaurat, etc..

Die Menge an Katalysator beträgt in der Regel 0,1 bis 5%, bezogen auf kondensationsvernetzendes

2

Organosilikon.

Die additions- bzw. kondensationsvernetzende Organosilikone enthaltenden, vorzugsweise in Pastenform vorliegenden Zusammensetzungen enthalten einen erheblichen Anteil an vorzugsweise mineralischen Füllstoffen wie Quarzmehl, kolloide Siliciumdioxide, Calciumsilikat, Calciumcarbonat, Calciumsulfat, Aluminiumoxid und Aluminiumhydroxid, etc.. Es können selbstverständlich auch Gemische verschiedener Füllstoffe eingesetzt werden.

Eine Übersicht über die Zusammensetzung und Anwendung von dentalen Silikon-Abformmassen findet sich in der Monographie von H. J. Rehberg, "Die Quintessenz der zahnärztlichen Abformhilfsmittel" (2. Auflage, 1978), insbesondere S. 70-83.

Der überraschende Effekt der erfindungsgemäß zusammengesetzten Silikon-Abdruckmassen wird durch die im folgenden beschriebenen Vergleichsversuche eindrucksvoll bestätigt, die zeigen, daß beim Einsatz von Aluminiumstearat eine optimale strukturviskose Einstellung der Masse möglich ist, während mit anderen mehrwertigen Metallsalzen wie Calcium-, Zink- und Magnesiumstearat dieser Effekt nicht erreicht werden konnte.

Eine Grundrezeptur der folgenden Zusammensetzung

| Christobalit | 36,5 (Gew.-%) |
| Calciumcarbonat | 3,0 |
| Kolloidales Siliciumdioxid | 1,0 |
| α,w-Dihydroxypolydimethylsiloxan | 50,0 |
| Polydimethylsiloxan | 7,5 |
| Stearinsäuresalz | 2,0 |

wurde auf ihr Viskositätserhalten untersucht.

Als Stearinsäregalze wurden alternativ Aluminiumtristearat, Zinkdistearat, Calciumdistearat und Magnesiumdistearat eingesetzt und die Viskositäten der jeweiligen Zusammensetzungen bestimmt.

Die Ergebnisse waren:

| | Al-tristearat | Zn-distearat | Ca-distearat | Mg-distearat |
|---|---|---|---|---|
| Viskosität bei 32 U/min. | 16.900 mPas | 14.400 mPas | 14.600 mPas | 14.400 mPas |
| Viskosität bei 8 U/min. | 34.500 mPas | 18.100 mPas | 18.100 mPas | 16.500 mPas |

Dies beweist, daß überraschenderweise eine befriedigende Einstellung der Strukturviskosität nur mit dem Aluminium-Fettsäuresalz möglich war.

Im folgenden werden einige Beispiele für erfindungsgemäß zusammengesetzte dentale Abformmassen gegeben, die nach Zusatz einer Katalysator und Vernetzer enthaltenden Zusammensetzung auf übliche Weise ausgehärtet werden können.

A. Kondensationsvernetzende Silikon-Abformmassen

|  | Beispiel 1<br>Gew.-% | Beispiel 2<br>Gew.-% |
|---|---|---|
| α,w-Dihydroxy-polydimethyl-siloxan | 45,0 | 45,0 |
| Polydimethylsiloxan | 12,5 | 8,0 |
| Stärke | - | 8,0 |
| Calciumcarbonat | 3,0 | 3,0 |
| Quarzmehl | 36,0 | 30,0 |
| Aluminiumstearat-Gemisch | 2,0 | 2,0 |
| Kolloidales Siliciumdioxid | 1,0 | 2,0 |
| Farbstoff | 0,5 | 2,0 |

Die Pasten können durch Vermischung mit Härterpaste oder -flüssigkeit auf übliche Weise ausgehärtet werden.

B. Additionsvernetzende Silikon-Abformmassen

4

EP 0 212 144 B1

| | Beispiel 1 | | Beispiel 2 | |
|---|---|---|---|---|
| | Basis-Paste Gew.-% | Katalysator-paste | Basis-paste | Katalysator-paste |
| Vinylendgestopptes Polydimethylsiloxan | 45,0 | 50,0 | 30,0 | 42,0 |
| Dimethylhydrogensylylendgestopptes Polydimethylsiloxan | 5,0 | - | 10,0 | - |
| Pt-Siloxan-Komplex | - | 0,02 | - | 0,01 |
| Aluminiumstearat (Mono-, Di-, Tristearat) | 2,0 | 2,0 | 1,5 | 1,5 |
| Quarzmehl | 40,0 | 40,0 | 50,0 | 50,0 |
| Calciumcarbonat | 5,5 | 5,48 | 8,0 | 5,99 |
| Kolloidales Siliciumdioxid | 2,0 | 2,0 | - | - |
| Farbstoff | 0,5 | 0,5 | 0,5 | 0,5 |

Beim Zusammenbringen beider Pasten erfolgt in bekannter Weise die Aushärtung.

**Patentansprüche**

1. Dentale Abformmasse auf Basis additions- oder kondensationsvernetzender Organosilikone, gekennzeichnet durch einen Gehalt von 0,1 bis 10 Gew.-%, bezogen auf die Gesamtzusammensetzung, an Aluminiumsalzen von $C_{12}$-$C_{18}$-Fettsäuren.

2. Dentale Abformmasse nach Anspruch 1, dadurch gekennzeichnet, daß sie die Aluminiumsalze der $C_{12}$-$C_{18}$-Fettsäuren in einer Menge von 0,5 bis 5 Gew.-%, bezogen auf die Gesamtzusammensetzung, enthält.

**3.** Dentale Abformmasse nach Anspruch 1 oder 2, gekennzeichnet durch einen Gehalt an Aluminiummono-, di-, und/oder -tristearat.

## Claims

**1.** Dental impression material based on addition-cross-linking or condensation-cross-linking organosilicones,
characterised by a content of 0.1 to 10% by weight, based on the total composition, of aluminium salts of $C_{12}$-$C_{18}$ fatty acids.

**2.** Dental impression material according to claim 1,
characterised in that it contains the aluminium salts of the $C_{12}$-$C_{18}$ fatty acids in a quantity of 0.5 to 5% by weight, based on the total composition.

**3.** Dental impression material according to claim 1 or 2,
characterised by a content of aluminium monostearate, distearate and/or tristearate.

## Revendications

**1.** Composition pour la prise d'empreinte dentaire à base d'organosilicones réticulant par addition ou par condensation, caractérisée par une teneur en sels d'aluminium d'acides gras en $C_{12}$ à $C_{18}$ de 0,1 à 10% en poids par rapport à la composition totale.

**2.** Composition pour la prise d'empreinte dentaire selon la revendication 1, caractérisée en ce qu'elle contient les sels d'aluminium d'acides gras en $C_{12}$ à $C_{18}$ en une quantité de 0,5 à 5% en poids par rapport à la composition totale.

**3.** Composition pour la prise d'empreinte dentaire selon la revendication 1 ou 2, caractérisée en ce qu'elle contient du mono-, du di- et/ou du tristéarate d'aluminium.